# EUROPEAN PATENT APPLICATION

(11) **EP 4 663 188 A1**
(43) Date of publication of application: **17.12.2025**
(21) Application number: 24926512.5
(22) Date of filing: 15.03.2024
(51) Int. Cl.: A61K 31/357, A61K 36/28, A61P 1/16, A61P 29/00, A61P 25/00, A61P 25/14, A61P 25/16

(54) **USE OF SILYBUM MARIANUM PHARMACEUTICAL COMPOSITION IN PREPARATION OF DRUGS FOR TREATING FATTY LIVER DISEASES**

(30) Priority: 28.02.2024 CN 202410223526
(71) Applicant: Health and Happiness (H&H) Hong Kong Limited, Quarry Bay, Hong Kong 999077 (HK)
(72) Inventor: ZHAO, Lingling, Guangzhou, Guangdong 510000 (CN); ZHANG, Hua, Guangzhou, Guangdong 510000 (CN); XIANG, Lan, Guangzhou, Guangdong 510000 (CN); YI, Meijuan, Guangzhou, Guangdong 510000 (CN); HUANG, Jingya, Guangzhou, Guangdong 510000 (CN); WANG, Lianjing, Guangzhou, Guangdong 510000 (CN); LIU, Wei, Guangzhou, Guangdong 510000 (CN)
(74) Representative: WSL Patentanwälte Partnerschaft mbB
(86) International application number: PCT/CN2024/081814
(87) International publication number: WO 2025/179637

(57) **Abstract**

The present application relates to use of a milk thistle pharmaceutical composition in preparation of a medicament for treating fatty liver diseases, and belongs to the technical field of medicines. The present application provides use of the pharmaceutical composition in preparation of a medicament for preventing and/or treating fatty liver disease, the active ingredient of the pharmaceutical composition comprises a 7-KDCA regulator; the 7-KDCA regulator comprises silymarin, or the 7-KDCA regulator comprises silybin, silychristin, silydianin and/or isosilybin; the silybin comprises silybin A and/or silybin B; and the isosilybin comprises isosilybin A and/or isosilybin B. Researches show that the pharmaceutical composition can effectively and safely treat fatty liver diseases in a low dosage, has anti-inflammatory activity, also has nerve growth factor activity, and has great application prospects in preparation of medicaments for preventing and/or treating fatty liver disease, resisting inflammation and preventing and/or treating senile dementia.

## Description

### CROSS REFERENCE TO RELATED APPLICATIONS

The present application claims priority to Chinese Patent Application No. 202410223526.X, entitled "USE OF A MILK THISTLE PHARMACEUTICAL COMPOSITION IN THE PREPARATION OF A MEDICAMENT FOR TREATING FATTY LIVER DISEASE", and filed to China National Intellectual Property Administration on February 28, 2024, the entire contents of which are incorporated herein by reference.

### TECHNICAL FIELD

The present application relates to use of a milk thistle pharmaceutical composition in the preparation of a medicament for treating fatty liver disease, and belongs to the technical field of medicaments.

### BACKGROUND

Metabolic dysfunction associated fatty liver disease (MASLD), formerly known as non-alcoholic fatty liver disease (NAFLD), is a global liver disease that affects at least 25% of the population in the world. MASLD can progress from simple steatosis (lipid accumulation) to metabolic dysfunction associated steatohepatitis (MASH), and further to liver fibrosis, cirrhosis and liver cancer. The pathogenesis of MASLD is multifactorial, involving genetic, metabolic and environmental factors. A key feature of MASLD is chronic liver inflammation, which plays a crucial role in the progression of the disease from simple steatosis to MASH and the subsequent. The production of proinflammatory cytokines, such as tumor necrosis factor (TNF) and interleukin-6 (IL-6), can promote the inflammation of MASLD. Another important factor affecting MASLD is the gut-liver axis, which mediates a bidirectional cycle between the intestinal flora and the liver; alterations in the intestinal flora, increased intestinal permeability, and the resulting excessive influx of microbial metabolites (such as lipopolysaccharide (LPS)) into the liver contribute to the production of TNF and interleukin-1β (IL-1β), leading to further progression of liver inflammation and MASLD.

Bile acid (BA) metabolism regulated by intestinal microbiota is also closely related to MASLD. Microorganisms containing bile salt hydrolase (BSH) can dissociate conjugated bile acids into free bile acids, and some primary bile acids can also be converted into secondary bile acids by intestinal flora. Therefore, the composition of BA is affected by changes in intestinal microbiota. As the endogenous ligand of farnesoid X receptor (FXR), bile acid not only affects lipid absorption, but also regulates glucose metabolism, lipid metabolism and energy metabolism by regulating the FXR signal pathway, playing a role in MASLD. Therefore, many medicaments used clinically to treat MASLD are FXR receptor agonists, such as obeticholic acid, Vonafexor, PX-104 and TERN-101. However, at present, these medicaments still have some problems. For example, medicaments related to FXR receptor agonists are in the clinical verification stage and have not passed clinical trials. As the world's first FXR receptor agonist-related medicament to enter phase III clinical trials, obeticholic acid still has the risk of causing medicament-induced liver injury. Therefore, there is an urgent need to find therapeutic medicaments for MASLD with good therapeutic effects, clear mechanisms of action and few side effects.

### SUMMARY OF THE INVENTION

In order to solve the above problems, the present application provides use of a pharmaceutical composition in the preparation of a medicament, wherein the medicament has any one of the following functions:
(a) prevention and/or treatment of fatty liver disease;
(b) anti-inflammatory; and
(c) prevention and/or treatment of neurodegenerative disease;
the active ingredient of the pharmaceutical composition comprises a 7-KDCA regulator; the 7-KDCA regulator comprises silymarin, or the 7-KDCA regulator comprises silybin, silychristin, silydianin and/or isosilybin; the silybin comprises silybin A and/or silybin B; and isosilybin comprises isosilybin A and/or isosilybin B. Silymarin is a natural active substance extracted from the seeds of milk thistle (*Silybummarianum (L.) Gaertn*.), mainly containing flavonolignans (silybin, silychristin, silydianin and isosilybin) and dihydroquercetin. Silybin (silybin A and silybin B) is the main active ingredient of silymarin, accounting for 50% to 70% of silymarin.

In one embodiment of the present application, the 7-KDCA regulator is silymarin, or the 7-KDCA regulator consists of silybin and isosilybin A; and the silybin consists of silybin A and silybin B.

In one embodiment of the present application, the mass ratio of silybin to isosilybin A in the medicament is (7 to 8):1.

In one embodiment of the present application, the fatty liver disease comprises metabolic dysfunction associated fatty liver disease (MAFLD).

In one embodiment of the present application, the treatment of fatty liver disease comprises treating fatty liver disease by regulation of bile acid metabolism; the regulation of bile acid metabolism comprises reducing the binding of 7-KDCA to the farnesol X receptor by lowering the level of 7-KDCA, thereby affecting the stability of the farnesol X receptor.

In one embodiment of the present application, the treatment of fatty liver disease comprises alleviation of inflammation of patients with fatty liver disease, reduction of liver weight of patients with fatty liver disease, reduction of fat weight of patients with fatty liver disease, reduction of liver weight/body weight ratio of patients with fatty liver disease, improvement of liver fatty degeneration of patients with fatty liver disease, reducing lipid droplet accumulation of patients with fatty liver disease, improving serum indicators of patients with fatty liver disease, improvement of liver function indicators of patients with fatty liver disease and/or improvement of intestinal flora of patients with fatty liver disease.

In one embodiment of the present application, the alleviation of inflammation of patients with fatty liver disease comprises alleviation of inflammation of patients with fatty liver disease by regulating the TLR4/NF-κB signal pathway; the improvement of intestinal flora of patients with fatty liver disease comprises regulating the relative abundance of intestinal flora that produces short-chain fatty acids in the intestine of patients with fatty liver disease, regulating the relative abundance of intestinal flora that produces secondary bile acids (second bile acid) in the intestine of patients with fatty liver disease and/or regulating the relative abundance of intestinal flora that contains bile salt hydrolase activity in the intestine of patients with fatty liver disease.

In one embodiment of the present application, the anti-inflammatory comprises alleviation inflammation in patients with inflammation by regulating the TLR4/NF-κB signal pathway.

In one embodiment of the present application, the treatment of neurodegenerative disease comprises treating neurodegenerative disease with the medicament as a nerve growth factor-like active substance.

In one embodiment of the present application, the neurodegenerative disease comprises Alzheimer's disease.

In one embodiment of the present application, the medicament further contains a pharmaceutical excipient.

In one embodiment of the present application, the pharmaceutical excipient comprises a stabilizer, a preservative, a disintegrant, a sweetener, a glidant and/or a diluent.

In one embodiment of the present application, the dosage form of the medicament is capsule, injection, powder, granule, paste and/or tablet.

In one embodiment of the present application, the ingredient of the nerve growth factor-like active substance comprises a 7-KDCA regulator; the 7-KDCA regulator comprises silymarin, or the 7-KDCA regulator comprises a silybin, a silychristin, a silydianin and/or a isosilybin; the silybin comprises silybin A and/or silybin B; and the isosilybin comprises isosilybin A and/or isosilybin B.

In one embodiment of the present application, the 7-KDCA regulator is silymarin, or the 7-KDCA regulator consists of silybin and isosilybin A; and the silybin consists of silybin A and silybin B.

In one embodiment of the present application, the mass ratio of silybin to isosilybin A in the medicament is (7 to 8):1.

The present application also provides use of a pharmaceutical composition in the preparation of a nerve growth factor active substance, wherein the ingredient of the nerve growth factor-like active substance comprises a 7-KDCA regulator; the 7-KDCA regulator comprises silymarin, or the 7-KDCA regulator comprises a silybin, a silychristin, a silydianin and/or a isosilybin; the silybin comprises silybin A and/or silybin B; and the isosilybin comprises isosilybin A and/or isosilybin B.

In one embodiment of the present application, the 7-KDCA regulator is silymarin, or the 7-KDCA regulator consists of silybin and isosilybin A; and the silybin consists of silybin A and silybin B.

In one embodiment of the present application, the mass ratio of silybin to isosilybin A in the medicament is (7 to 8):1.

The technical solution of the present application has the following advantages:
1. The present application provides use of a pharmaceutical composition in the preparation of a medicament for prevention and/or treatment of fatty liver disease, wherein the active ingredient of the pharmaceutical composition comprises a 7-KDCA regulator; the 7-KDCA regulator comprises silymarin, or the 7-KDCA regulator comprises silybin, silychristin, silydianin and/or isosilybin; the silybin comprises silybin A and/or silybin B; and the isosilybin comprises isosilybin A and/or isosilybin B.

Animal experiments have shown that the pharmaceutical composition provided by the present application can reduce inflammation of MASLD model mice, reduce liver weight of MASLD model mice, reduce fat weight of MASLD model mice, reduce liver weight/body weight ratio of MASLD model mice, improve liver fatty degeneration of MASLD model mice, reduce lipid droplet accumulation of MASLD model mice, improve serum indexes of MASLD model mice, improve liver function indicators of MASLD model mice, and improve intestinal flora of MASLD model mice (including regulating the relative abundance of intestinal flora producing short-chain fatty acids of MASLD model mice, regulating the relative abundance of intestinal flora producing secondary bile acids of MASLD model mice, and regulating the relative abundance of intestinal flora containing bile salt hydrolase activity of MASLD model mice, etc.), which has a good treatment for MAFLD. In addition, animal experiments have shown that the pharmaceutical composition provided by the present application can significantly improve the liver function indicators of MASLD model mice (including reducing the levels of triglycerides, total cholesterol, aminotransferase, aspartate aminotransferase and total bile acid in the serum of MASLD model mice), has a certain liver protection effect, and will not cause liver damage when used for the treatment of MAFLD.

It has been demonstrated by mechanism experiments that the pharmaceutical composition provided by the present application can reduce inflammation in MASLD model mice by regulating the TLR4/NF-κB signal pathway, and the pharmaceutical composition provided by the present application can reduce the level of 7-KDCA of MASLD model mice and thus reduce the binding of 7-KDCA to farnesoid X receptor of MASLD model mice (negative feedback regulation), thereby affecting the stability of farnesoid X receptor of MASLD model mice, so as to achieve the purpose of treating MASLD.

Therefore, the pharmaceutical composition provided by the present application can effectively and safely treat fatty liver disease (especially MAFLD), and has great application prospects in the preparation of medicaments for preventing and/or treating fatty liver disease (especially MAFLD).

Further, the 7-KDCA regulator consists of a silybin and isosilybin A; the silybin consists of silybin A and silybin B; the mass ratio of silybin to isosilybin A in the medicament is (7 to 8):1. It has been demonstrated by animal experiments that compared with high-dose silymarin (80 mg/kg/day silymarin), lower doses of silybin and isosilybin A (44.87 mg/kg/day silybin + 6.09 mg/kg/day isosilybin A) can effectively treat MASLD, and the effect is close to that of high-dose silymarin (80 mg/kg/day silymarin). Therefore, the pharmaceutical composition provided by the present application can effectively and safely treat fatty liver disease (especially MAFLD), and has great application prospects in the preparation of medicaments for preventing and/or treating fatty liver disease (especially MAFLD).

2. The present application provides use of a pharmaceutical composition in the preparation of a medicament for anti-inflammatory, wherein the active ingredient of the pharmaceutical composition comprises a 7-KDCA regulator; the 7-KDCA regulator comprises silymarin, or the 7-KDCA regulator comprises a silybin, a silychristin, a silydianin and/or a isosilybin; the silybin comprises silybin A and/or silybin B; and the isosilybin comprises isosilybin A and/or isosilybin B. Cell experiments have shown that the pharmaceutical composition provided by the present application can exhibit excellent anti-inflammatory activity at the cellular level, animal experiments have shown that the pharmaceutical composition provided by the present application can reduce inflammation of MASLD model mice, and mechanism experiments have shown that the pharmaceutical composition provided by the present application can reduce inflammation of MASLD model mice by regulating the TLR4/NF-κB signal pathway. Therefore, the pharmaceutical composition provided by the present application has great application prospects in the preparation of anti-inflammatory medicaments.

3. The present application provides use of a pharmaceutical composition in the preparation of a medicament for prevention and/or treatment of neurodegenerative disease, wherein the active ingredient of the pharmaceutical composition comprises a 7-KDCA regulator; the 7-KDCA regulator comprises silymarin, or the 7-KDCA regulator comprises silybin, silychristin, silydianin and/or isosilybin; the silybin comprises silybin A and/or silybin B; and the isosilybin comprises isosilybin A and/or isosilybin B. Alzheimer's disease is a stubborn disease that seriously threatens the health of the elderly. The medicaments currently on the market for treating neurodegenerative diseases such as Alzheimer's disease only play a role in alleviating symptoms, and it is still an incurable disease. It is reported that the onset of neurodegenerative diseases such as Alzheimer's disease is related to nerve cell damage and death, and nerve growth factor (NGF) has an important effect on the growth, survival and functional maintenance of neurons. Since NGF is highly hydrophilic and has a large molecular weight, it cannot pass through the blood-brain barrier, which limits its application in the treatment of neurodegenerative diseases such as Alzheimer's disease. Therefore, it has become a research hotspot to find small molecule compounds with NGF-like activity that can pass through the blood-brain barrier smoothly and have the activity of NGF. Cell experiments have shown that the pharmaceutical composition provided by the present application has the activity of NGF-like activity, and each component in the pharmaceutical composition is a small molecule compound. Therefore, the pharmaceutical composition provided by the present application, as a NGF-like active substance, has great application prospects in the preparation of medicaments for preventing and/or treating neurodegenerative diseases such as Alzheimer's disease.

### BRIEF DESCRIPTION OF THE DRAWINGS

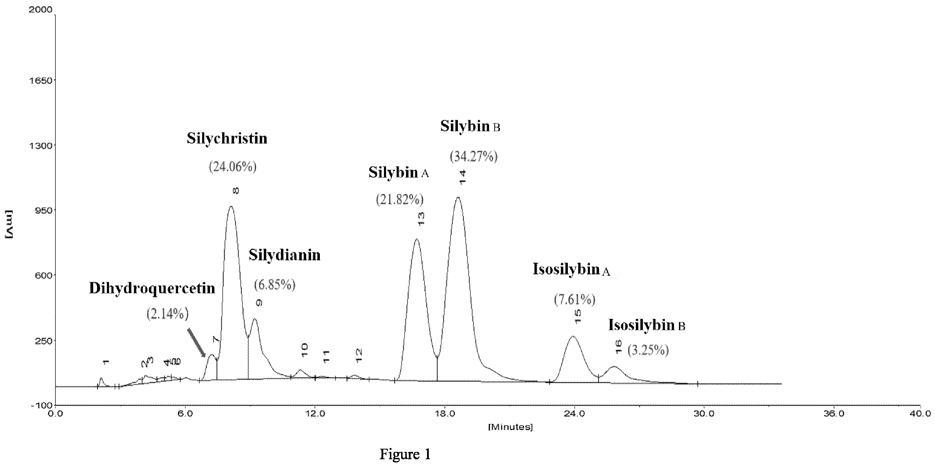

Figure 1: HPLC analysis results of silymarin.

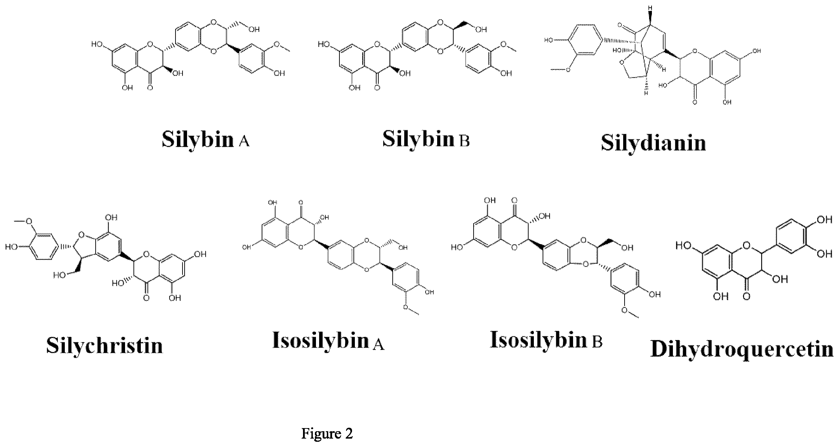

Figure 2: Chemical structures of the main components of silymarin.

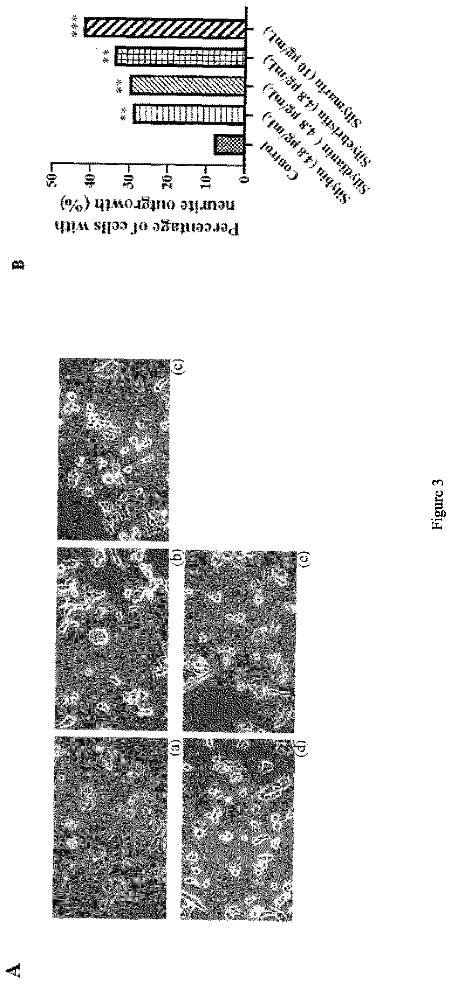

Figure 3: Effects of silymarin and its main components on PC12 cells. In Figure 3, (A) PC12 cell images after sample treatment, in (A), (a) negative control, (b) silybin (4.8µg/mL), (c) silydianin (4.8µg/mL), (d) silychristin (4.8µg/mL), (e) silymarin (4.8µg/mL); (B) quantification results of Figure A.

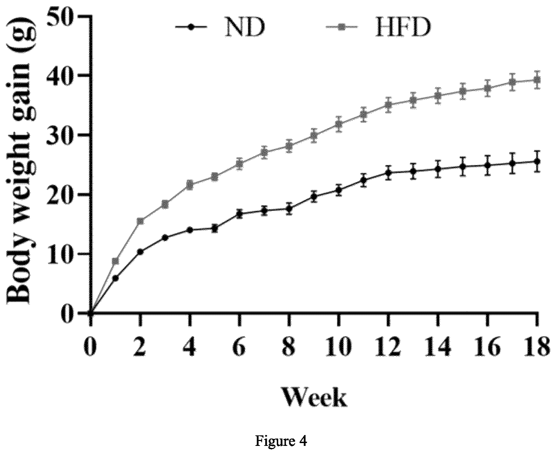

Figure 4: Body weight changes of mice during high-fat diet modeling.

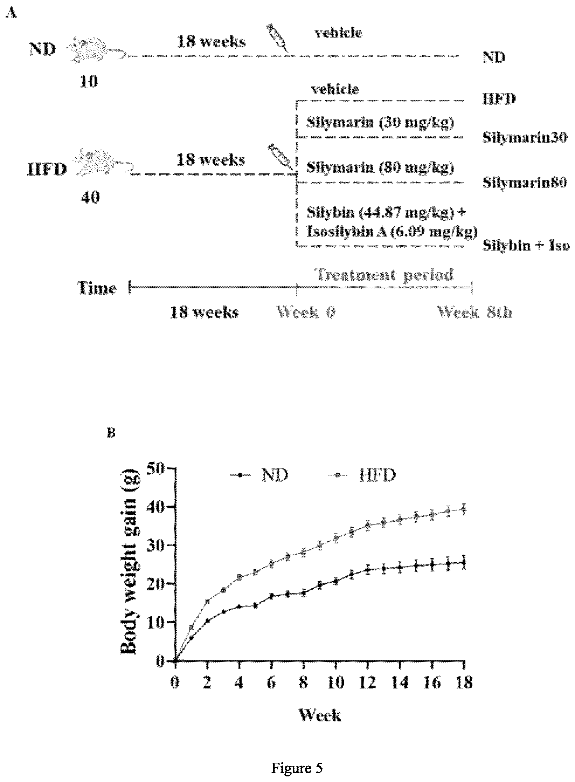

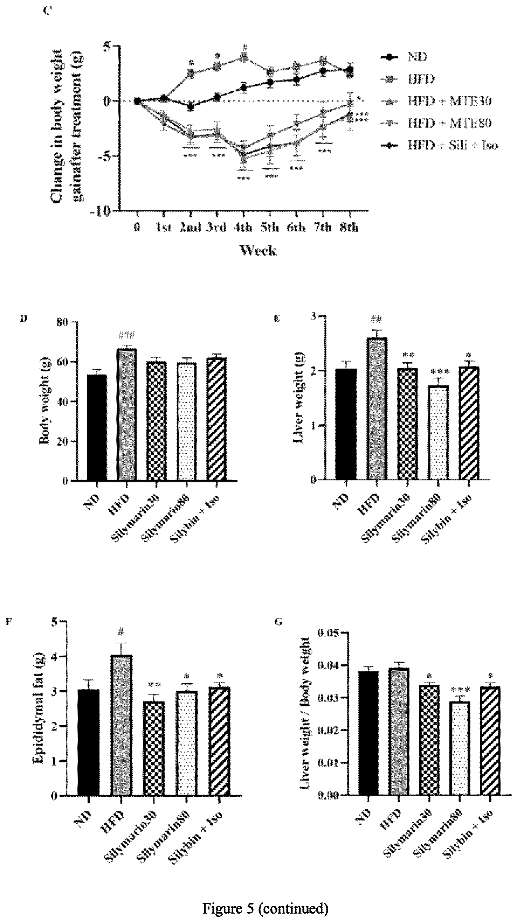

Figure 5: Effects of silymarin and its main components on body weight, liver weight and fat weight of HFD-fed mice. In Figure 5, (A) design of drug dosing protocol for mice; (B) changes in mice body weight before dosing; (C) changes in mice body weight during dosing; (D) mice body weight after dosing; (E) mice liver weight after dosing; (F) mice epididymal fat weight after dosing; (G) mice liver weight/body weight ratio after dosing.

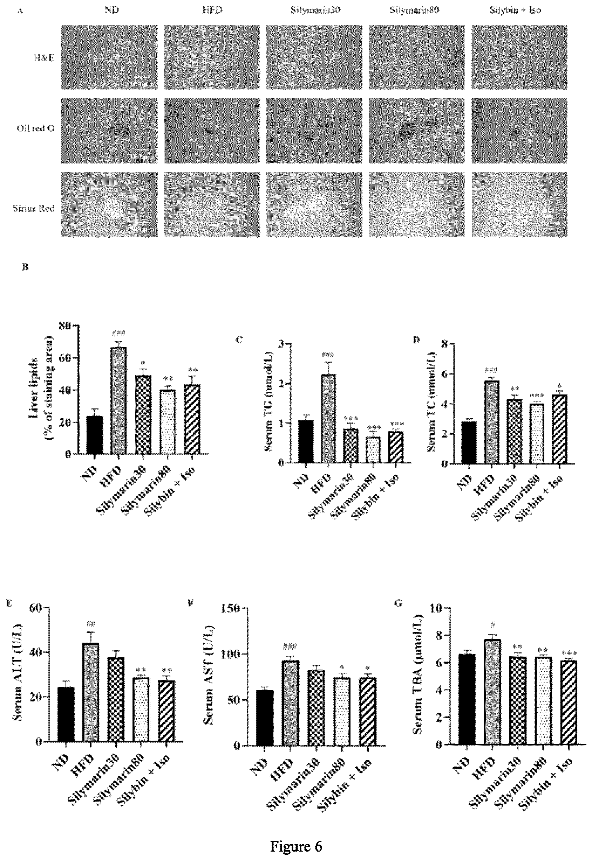

Figure 6: Effects of silymarin on liver steatosis, serum biochemical indexes and liver function indicators of HFD mice. In Figure 6, (A) mice liver histological staining; (B) mice liver oil red staining quantification results; (C) mice serum triglyceride level; (D) mice serum total cholesterol level; (E) mice serum ALT level; (F) mice serum AST level; (G) mice serum total bile acid level.

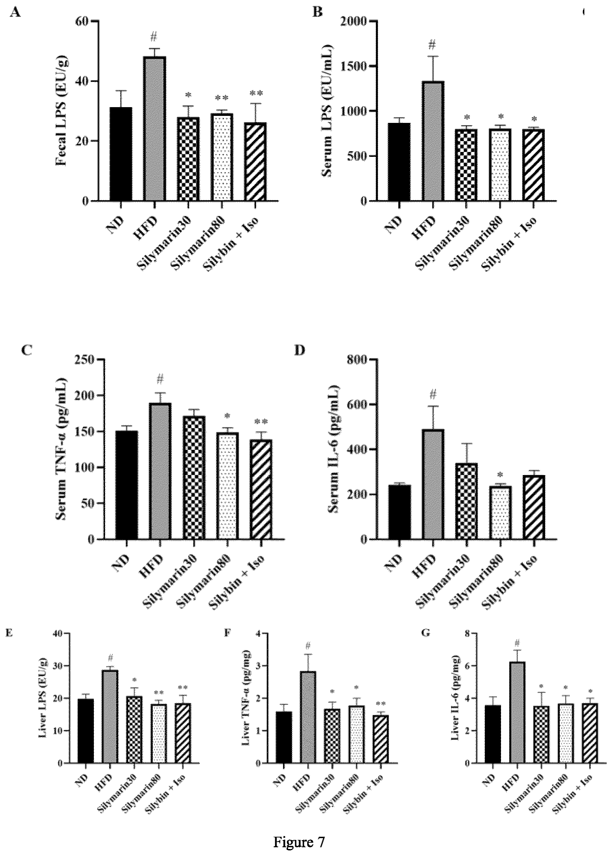

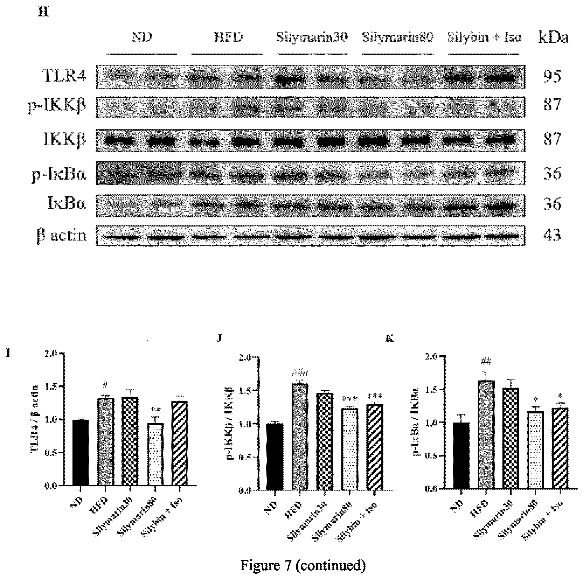

Figure 7: Effect of silymarin on inflammation of HFD mice. In Figure 7, (A) LPS level in mice feces; (B) LPS level in mice serum; (C) TNF-α level in mice serum; (D) IL-6 level in mice serum; (E) LPS level in mice liver; (F) TNF-α level in mice liver; (G) IL-6 level in mice liver; (H) WB results of TLR4, p-IKKβ, IKKβ, p-IκBα, IκBα, and β actin in mice liver; (I) quantification results of TLR4/β actin; (J) quantification results of p-IKKβ/IKKβ; (K) quantification results of p-IκBα/IκBα.

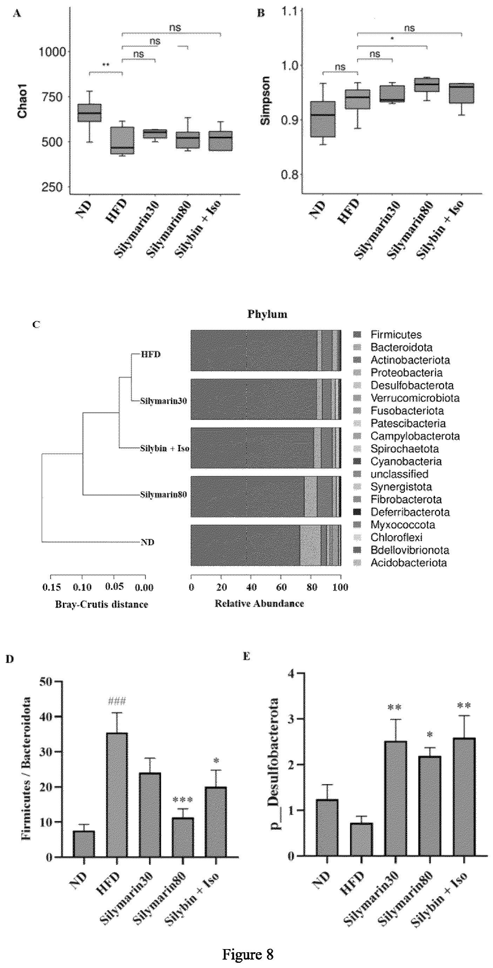

Figure 8: Effects of silymarin on intestinal flora of HFD mice. In Figure 8, (A) α diversity analysis-Chaol index; (B) α diversity analysis-Simpson index; (C) Venn diagram; (D) PCoA analysis; (E) phylum level clustering analysis; (F) phylum level, relative abundance of intestinal flora; (G) *Firmicutes* / *Bacteroidota* (F/B); (H) relative abundance of *Desulfobacterota.*

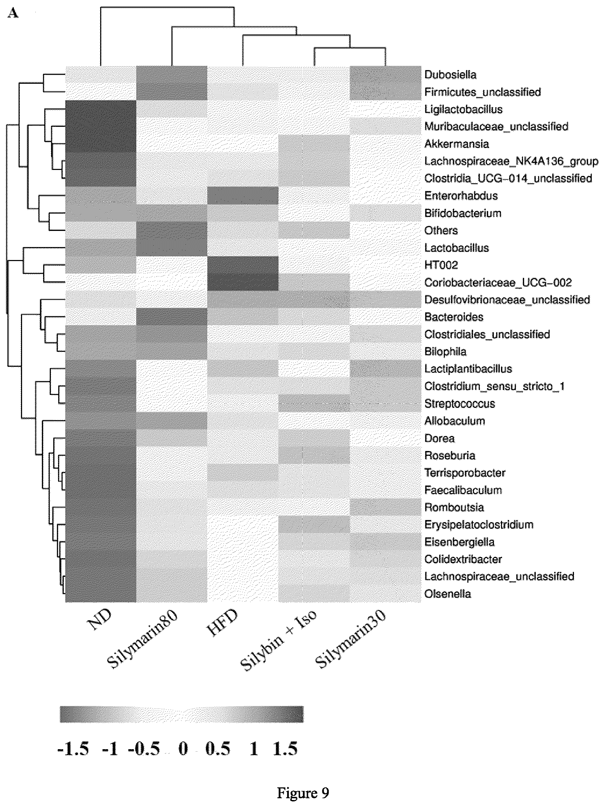

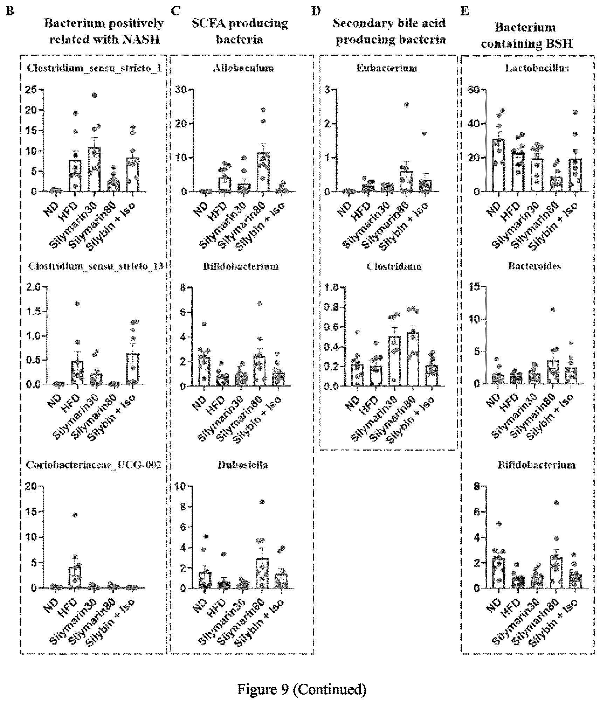

Figure 9: Effect of silymarin on the relative abundance of intestinal flora at the genus level of HFD mice. In Figure 9, (A) clustering diagram (heat map) of intestinal flora at the genus level; (B) relative abundance of intestinal flora positively correlated with NASH; (C) relative abundance of intestinal flora producing SCFA; (D) relative abundance of intestinal flora producing secondary bile acid; (E) relative abundance of intestinal flora containing BSH.

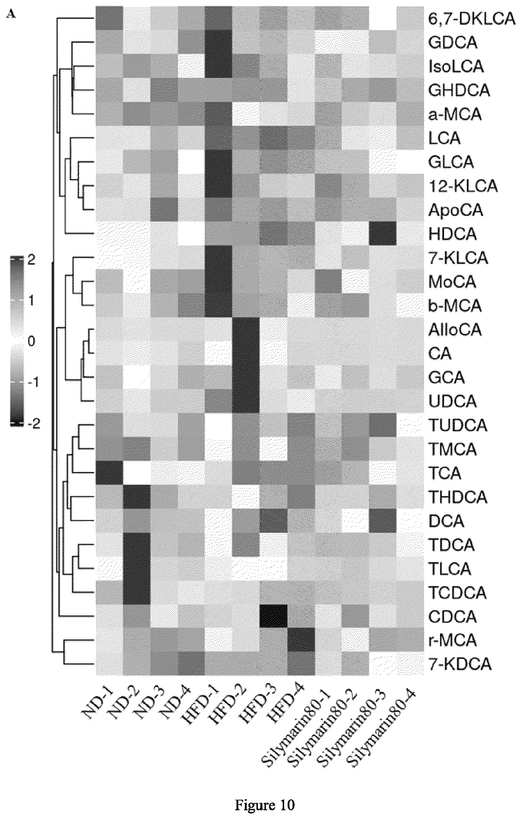

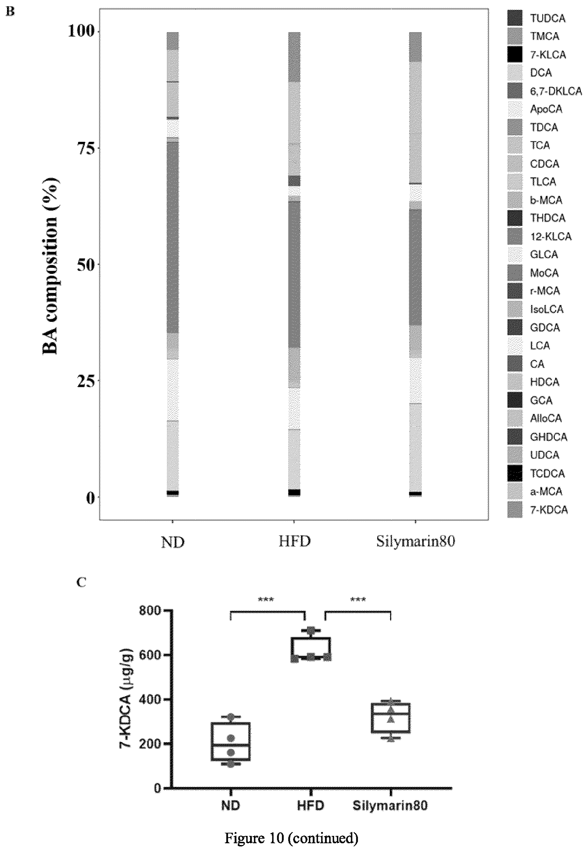

Figure 10: Effect of silymarin on bile acid metabolism of HFD mice. In Figure 10, (A) heat map of targeted metabolic analysis of bile acid in mice feces; (B) composition and content ratio of bile acid in mice feces; (C) 7-KDCA level.

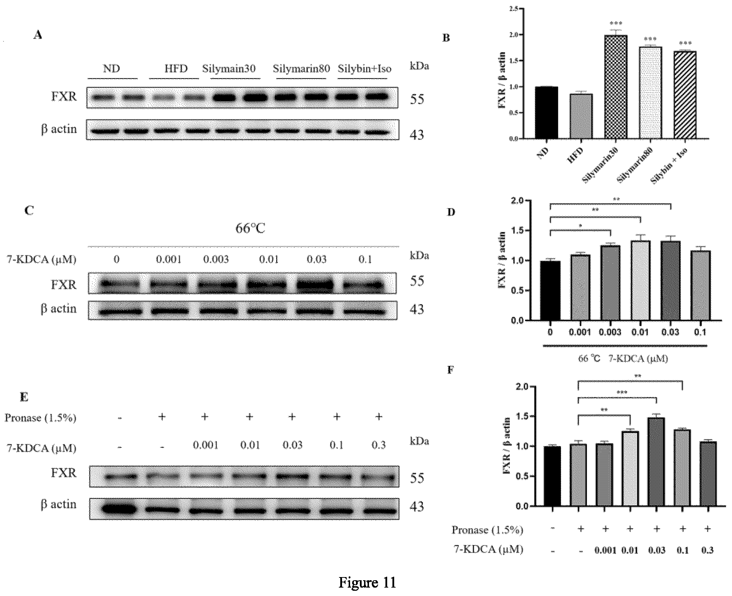

Figure 11: FXR expression level and the effect of 7-KDCA on FXR stability. In Figure 11, (A) WB analysis results of mice liver FXR; (B) FXR/β actin quantification results; (C) CETSA results of 7-KDCA and FXR; (D) quantification analysis results of Figure A; (E) DARTS results of 7-KDCA and FXR; (F) quantification analysis results of Figure C.

### DETAILED DESCRIPTION

The following examples are provided for a better understanding of the present application, and are not limited to the best embodiments, and do not limit the content and protection scope of the present application. Any product identical or similar to the present application obtained by combining with the features of other prior art shall fall within the protection scope of the present application.

Some specific experimental steps or conditions are not indicated in the examples can be carried out according to the operation or conditions of conventional experimental steps described in the literature in this field. If the manufacturer of the reagent or instrument used is not indicated, it is a conventional reagent product commercially available.

### Experimental Example 1: Analysis of the main chemical components of silymarin

The experimental process is as follows:
In order to clarify the main chemical components of silymarin, silymarin samples (purchased from Zhejiang Huisong Pharmaceutical Co., Ltd.) were studied. Silymarin as well as silybin, silychristin, silydianin and other standards were analyzed by HPLC (Cosmosil 5C18-MS-II Packed Column (Φ4.6/250mm), flow rate 1mL/min, detection wavelength: 210nm, 50% methanol, 60min), and area normalization calculation was performed to determine the main components and content proportions of silymarin: silybin (A, B, 56.09%, t_{R} =27.30min, 31.57min), silychristin (24.06%, t_{R} =9.99min), silydianin (6.85%, t_{R} =12.03min), isosilybin A (7.61%, t_{R} =45.70min), isosilybin B (3.25%, t_{R} = 50.63min) and dihydroquercetin (2.14%, t_{R} =6.95min), and their retention times were basically consistent with those of the standard. The HPLC analysis results are shown in Figure 1 and the structure of the main components of silymarin is shown in Figure 2.

### Experimental Example 2: Experiment on the nerve growth factor-like active of Silymarin and its main components

The experimental process is as follows:
PC12 cells (purchased from the Chinese Academy of Sciences Cell Bank) were added to a 24 well-plate with 1 mL DMEM culture medium (Dublecco modified Eagle culture medium, purchased from Thermo Scientific) containing 10% (v/v) fetal bovine serum, 5% (v/v) horse serum and 1% (w/v, g/100mL) premixed antibiotics (purchased from Invitrogen) with tan amount of 2×10⁵ CFU/well. The 24-well plates were cultured in a cell incubator at 37°C and 5% (v/v) CO₂ for 24h. After culture, fresh DMEM medium was changed, and the 24-well plates were divided into negative control group, silybin group, silydianin group, silychristin group and silymarin group, 3 duplicated wells for each group. After the end of the grouping, silybin group, silydianin group, silychristin group and silymarin group were added to the final concentration of 4.8 µg/mL, respectively, while no substance was added to the negative control group. Among them, silybin was purchased from Chengdu Herb Substance, silychristin and silydianin were purchased from Chengdu ABPHYTO Biotechnology, and silymarin was purchased from Zhejiang HUISONG Pharmaceutical Co., Ltd. After the addition of the sample, the 24-well plate was cultured in a cell incubator at 37°C and 5% (v/v) CO₂ for 24h; after the culture, the morphological changes of the cell morphology were observed under a microscope and the cell process differentiation rate (cell neurite differentiation rate = the number of cells with neurites longer than one time the longest diameter of the cell body/the total number of cells in the selected field of view × 100%) was recorded. The results showed that compared with the control group, silybin and its main components significantly increased the proportion of PC12 cells with neurites (A in Figure 3 and B in Figure 3), indicating that silybin and its main components all have nerve growth factor-like activity.

### Experimental Example 3: Effects of silymarin and its main components on MASLD model mice

The experimental process is as follows:

### 1. Evaluation of the efficacy of silymarin and its main components

### 1.1. Establishment of MASLD mice model using high-fat diet

Fifty ICR male mice (four weeks old) were purchased from Hangzhou Medical College and fed in the Experimental Animal Center of Zhejiang University (the feeding conditions were room temperature 23°C, 55% humidity, and 12h light/dark cycle). After one week of adaptive feeding of mice, the mice were divided into a normal feed group (ND group, n=10) and a high-fat feed group (HFD group, n=40). The normal feed group was fed with normal feed, and the high-fat feed group was fed with high-fat feed for MASLD modeling, and the weight changes of mice were recorded weekly. The normal feed was provided by the Experimental Animal Center of Zhejiang University, and the high-fat feed was purchased from Nantong Trofi Feed Technology Co., Ltd. (feed code TP23300, containing 60% fat, 20.6% carbohydrates and 19.4% protein). After 18 weeks of modeling, there was a significant difference in the weight of mice in the ND group and the HFD group. The average weight gain of mice in the ND group was 25.6g, while the average weight gain of mice in the HFD group was 39.3g (Figure 4).

### 1.2 Effects of silymarin and its main components on liver weight, fat weight and liver weight/body weight ratio of HFD-induced MASLD model mice

After 18 weeks of modeling, the MASLD model mice were randomly divided into four groups: HFD group (given high-fat diet and water), Silymarin30 group (given high-fat diet and 30 mg/kg/day silymarin), Silymarin80 group (given high-fat diet and 80 mg/kg/day silymarin) and Silybin+Iso group (given high-fat diet and 44.87 mg/kg/day silybin + 6.09 mg/kg/day isosilybin A) (as shown in A in Figure 5), 10 mice for each group, and continued intragastric administration for 8 weeks. Among them, the dose of silybin and isosilybin A was obtained by multiplying 80 mg/kg by the proportion of silybin and isosilybin A in silymarin (56.09%, 7.61%). Silybin and isosilybin A was purchased from Chengdu Herb Substance, and silymarin was purchased from Zhejiang HUISONG Pharmaceutical Co., Ltd. The changes in body weight in the ND and HFD groups during the treatment period are shown in Figure 5. After 8 weeks of administration, the body weight of the mice was recorded and then dissected, and the weights of the liver and epididymal fat were measured. The body weight, liver weight, and epididymal fat weight of the HFD group were significantly higher than those of the ND group (D and F in Figure 5, p<0.001, p<0.01, p<0.05). The HFD-induced weight gain was reduced among the treatment groups, but there was no significant difference (D in Figure 5). However, compared with the HFD group, the liver weight of the Silymarin30, Silymarin80, and Silybin+Iso treatment groups was significantly reduced (E in Figure 5, p<0.01, p<0.001, p<0.05), and the epididymal fat weight was also significantly reduced (F in Figure 5, p<0.01, p<0.05, p<0.05). The liver weight/body weight ratio showed no difference between the ND and HFD groups, but was significantly lower in the three treatment groups compared with the HFD group (G in Figure 5, p<0.05, p<0.001, and p<0.05, respectively). These results indicated that silymarin or the combination of silybin and isosilybin A significantly reduced HFD-induced increases in liver and fat weight and reduced the liver weight/body weight ratio in mice, with the Silymarin80 group having the best effect.

### 1.3 Effects of silymarin and its main components on hepatic steatosis, lipid droplet accumulation, and serum liver function indicators in HFD-induced MASLD model mice

After 8 weeks of intragastric administration, 3 mice liver samples were randomly selected from each group of mice for histological staining and observation under a microscope. Hematoxylin and eosin (H&E) staining was used to observe the morphology of hepatocytes. HE staining results showed that the HFD group had significant hepatic steatosis and liver damage, which could be improved by Silymarin30, Silymarin80 and Silybin+Iso (A in Figure 6). Then, Oil Red O staining was used to observe the formation of lipid droplets. The results showed that the HFD group had a large amount of lipid droplet accumulation (A and B in Figure 6, p<0.001), while the lipid droplet accumulation in the Silymarin30, Silymarin80 and Silybin+Iso groups was reduced (A and B in Figure 6, p<0.05, p<0.01, p<0.01). Sirius red and its contrast dye liquor are both strong acidic dyes, which are easily combined with the basic groups in collagen molecules. They are red under an optical microscope and can therefore be used to observe liver fibrosis. The results showed that a small amount of collagen fibers were observed in the HFD group, but the collagen fibers were reduced in the ND group and the treatment group (A in Figure 6), indicating that liver fibrosis was alleviated after silymarin treatment. In addition, after 8 weeks of intragastric administration, mice plasma was collected by orbital bleeding, and serum was obtained by centrifugation (12,000rpm centrifugation for 30min) after standing for half an hour. Serum samples (200µL) of 8 mice were randomly selected from each group to evaluate triglyceride (TG), total cholesterol (TC), aminotransferase (ALT), aspartate aminotransferase (AST) and total bile acid (TBA) levels (detection was completed by Hangzhou Haoke Biology). Compared with the ND group, the levels of triglyceride (TG) and total cholesterol (TC) in the HFD group were significantly increased (C and D in Figure 6, p<0.001, p<0.001), and the levels of TG and TC in each treatment group were significantly decreased (C in Figure 6, p<0.001, p<0.001, <0.01; D in Figure 6, p<0.01, p<0.001, p<0.05). The levels of alanine aminotransferase (ALT) and aspartate aminotransferase (AST), which reflect hepatocyte damage, were significantly increased in the HFD group, while they were significantly decreased in the Silymarin80 and Silybin+Iso groups (E in Figure 6, p<0.01, p<0.01; F in Figure 6, p<0.001, p<0.05, p<0.05). Serum total bile acid (TBA) can reflect the level of hepatocyte damage and hepatic excretion dysfunction. The results showed that the increase in serum TBA levels induced by HFD was reduced in each treatment group (G in Figure 6, p<0.05, p<0.01, p<0.01, p<0.001). These results indicated that silymarin or the combination of silybin and isosilybin A can not only alleviate HFD-induced hepatic steatosis and reduce serum TG and TC levels, but also improve liver function indicators.

### 1.4 Effects of silymarin and its main components on serum liver function indicators in HFD-induced MASLD model mice

In addition, fifty ICR male mice (four weeks old) were purchased from Hangzhou Medical College and fed in the Experimental Animal Center of Zhejiang University (the feeding conditions were room temperature 23°C, 55% humidity, and 12h light/dark cycle). After one week of adaptive feeding of mice, the mice were divided into a normal feed group (ND group, n=5) and a high-fat feed group (HFD group, n=45). The normal feed group was fed with normal feed, and the high-fat feed group was fed with high-fat feed for MASLD modeling, and the weight changes of mice were recorded weekly. The normal feed was provided by the Experimental Animal Center of Zhejiang University, and the high-fat feed was purchased from Nantong Trofi Feed Technology Co., Ltd. (feed code TP23300, containing 60% fat, 20.6% carbohydrates and 19.4% protein).

After 18 weeks of modeling, the MASLD model mice were randomly divided into nine groups: HFD group (given high-fat diet and water), silymarin group (given high-fat diet and 80 mg/kg/day silymarin), silybin 7 & isosilybin A1 group (given high-fat diet and 44.87 mg/kg/day silybin + 6.09 mg/kg/day isosilybin A), silybin 5 & isosilybin A5 group (given high-fat diet and 25.48 mg/kg/day silybin + 25.48 mg/kg/day isosilybin A), silybin group (given high-fat diet and 44.87 mg/kg/day silybin), isosilybin group (given high-fat diet and 6.09 mg/kg/day isosilybin), silydianin group (given high-fat diet and 5.48 mg/kg/day silydianin), silychristin group (given high-fat diet and 19.25 mg/kg/day silychristin) and dihydroquercetin group (given high-fat diet and 1.71 mg/kg/day dihydroquercetin) (as shown in Table 1), 5 mice for each group, and continued intragastric administration for 8 weeks. Among them, the dose of silybin 7 & isosilybin A1 group was obtained by the ratio of silybin to isosilybin A in silymarin, and the dose of silybin 5 & isosilybin A5 group was obtained by the total weight of silybin 7 & isosilybin A1 group. Silybin and isosilybin A were purchased from Chengdu Herb Substance, silychristin, silydianin and dihydroquercetin were purchased from Chengdu ABPHYTO Biotechnology, and silymarin was purchased from Zhejiang HUISONG Pharmaceutical Co., Ltd.

After 8 weeks of intragastric administration, mice plasma was collected by orbital bleeding, and serum was obtained by centrifugation (12,000rpm centrifugation for 30min) after standing for half an hour. Serum samples (200µL) of 5 mice were randomly selected from each group to evaluate triglyceride (TG), total cholesterol (TC), aminotransferase (ALT), aspartate aminotransferase (AST) and total bile acid (TBA) levels (detection was completed by Hangzhou Haoke Biology).The results further indicated that only silymarin or a specific ratio combination of silybin and isosilybin A could reduce serum TG and TC levels and improve liver function indicators (Table 1, * is p<0.05, ** is p<0.01, *** is p<0.01).

**Table 1 Levels of triglyceride, total cholesterol, aminotransferase and aspartate aminotransferase in serum of mice in different groups**

| Group | TG | sd | TC | sd | ALT | sd | AST | sd |
|---|---|---|---|---|---|---|---|---|
| HFD | 2.13±0.022 | | 5.62±0.169 | | 45.36±1.92 | | 90.36±3.88 | |

| ND | 1.02±0.006 | *** | 2.36+0.057 | *** | 25.36±1.12 | *** | 65.23+2.38 | *** |
|---|---|---|---|---|---|---|---|---|
| Silymarin | 0.63±0.079 | *** | 3.66±0.148 | *** | 28.36±2.85 | *** | 78.32+4.27 | *** |
| Silybin 7 & isosilybin A1 | 0.78+0.177 | *** | 4.41±0.062 | *** | 32.12±2.38 | *** | 90.46+5.09 | *** |
| Silybin 5 & isosilybin A5 | 1.95±0.112 | | 5.36+0.058 | | 40.36±1.69 | | 85.16+3.71 | |
| Silybin | 1.96±0.111 | | 5.55+0.071 | | 40.32±1.49 | | 92.36+3.84 | |
| Isosilybin | 2.02±0.037 | | 5.23+0.174 | | 41.34±2.77 | | 89.36+4.81 | |
| Silydianin | 2.05±0.076 | | 5.36+0.175 | | 42.09±1.21 | | 94.35+3.49 | |
| Silychristin | 1.94±0.147 | | 5.19±0.127 | | 39.69±1.88 | | 91.03±5.68 | |
| Dihydroquercetin | 2.09±0.134 | | 5.41±0.156 | | 39.86±2.03 | | 92.27±4.63 | |

### 2. Research on the mechanism of action of silymarin and its main components

### 2.1 Effects of silymarin and its main components on alleviating inflammation of HFD-fed mice by regulating the TLR4/NF-κB signal pathway

In order to explore whether the effects of silymarin and its main components are related to regulating inflammation, ELISA kits were used to detect the levels of pro-inflammatory cytokines in the feces, serum and liver of mice. For fecal samples, based on Part 1.2, 30 mg of mice feces collected after 8 weeks of intragastric administration were taken, and the LPS level was measured using an Elisa kit (purchased from Hefei Laier Biological). For liver and serum samples, based on Part 1.2, 30 mg of mice liver collected after dissection of mice administered with intragastric administration for 8 weeks was taken, and the levels of LPS (lipopolysaccharide), TNF-α (tumor necrosis factor) and IL-6 (interleukin-6) in the liver and serum of mice were measured using an Elisa kit (purchased from Suzhou Yikesai Biological). The results of mice fecal LPS levels showed that silymarin and Silybin+Iso reversed the increase in LPS levels in mice feces caused by HFD (A in Figure 7, p<0.05, p<0.05, p<0.01, p<0.01). HFD also led to increased levels of serum LPS, TNF-α, and IL-6 (from B to D in Figure 7, p<0.05, p>0.05, p<0.05). Among them, the serum LPS level of all treatment groups was significantly reduced (B in Figure 7, p<0.05, p<0.05, p<0.05), but only Silymarin80 and Silybin+Iso reduced the serum TNF-α level (C in Figure 7, p<0.05, p<0.01), and only Silymarin80 reduced the serum IL-6 level (D in Figure 7, p<0.05). The results of levels of pro-inflammatory cytokines in the liver showed that all treatment groups reduced the HFD-induced increase in liver LPS (E in Figure 7, p<0.05, p<0.05, p<0.01, p<0.01), TNF-α (F in Figure 7, p<0.05, p<0.05, p<0.05, p<0.01), and IL-6 (G in Figure 7, p<0.05, p<0.05, p<0.05, p<0.05) levels. The TLR4/NF-κB signal pathway can be activated by LPS and proinflammatory cytokines, so the expression levels of related proteins in this signal pathway in mice liver were evaluated using Western blot (WB) analysis. Based on Section 1.2, after 8 weeks of intragastric administration, liver samples of 3 mice were randomly selected from each group and mixed, 30 mg of mixed liver samples were taken, and the protein concentration of the mice liver protein solution was measured using a BCA kit (purchased from CoWin Biotech), and the samples to be tested were subjected to immunoblot analysis (Western blot). The results showed that the expression levels of TLR4 and phosphorylated IκBα were significantly increased in the HFD group (H, I and K in Figure 7, p<0.05, p<0.01), and were significantly reduced by Silymarin80 (H, I and K in Figure 7, p<0.01, p<0.05). The Silybin+Iso group significantly increased the phosphorylation level of IKKβ (H and J in Figure 7, p<0.05), but reduced the increase in the phosphorylation level of IκBα caused by HFD (H and K in Figure 7, p<0.01, p<0.05). These results indicated that silymarin reduced inflammatory signal pathways by regulating TLR4/NF-κB and that it is more effective than the combination of silybin and isosilybin A.

### 2.2 Silymarin regulates the intestinal flora composition of HFD-fed mice

The gut-liver axis plays an important role in the development of MASLD. To investigate whether silymarin changes the intestinal microbiota, based on section 1.2, after 8 weeks of intragastric administration, the mice cages were cleaned and disinfected with alcohol, and fecal samples of mice were collected. Fecal samples (2 pellets) of 8 mice were randomly selected from each group to extract DNA. According to the specified sequencing region, PE250 sequencing analysis was performed on the 16S rDNA V4 region of the sample using Hiseq 2500 instrument. The results of α-diversity analysis of the mice intestinal microbiota were shown in A and B in Figure 8. HFD reduced the total number of microorganisms in mice feces, and silymarin or Silybin+Iso did not restore the total number of microorganisms (A in Figure 8, p<0.01). HFD did not change the richness of microbial species in mice feces, but the species richness was significantly increased in the Silymarin80 group (B in Figure 8, p<0.05). To study the differences and similarities between different groups in more detail, cluster analysis was performed for each group at the phylum level (C in Figure 8). The results showed that the microbial composition of the Silymarin80 group and the ND group was the most similar, followed by the Silybin+Iso group and the Silymarin30 group. The composition difference between the HFD group and the ND group was the largest (C in Figure 8). These results indicate that the HFD altered the composition of the intestinal microbiota of mice, while 80 mg/kg silymarin partially restored it to the level of the ND group. An increase or decrease in the ratio (F/B) of *Firmicutes* and *Bacteroidota* is generally considered to be intestinal dysbiosis, where an increase in F/B is generally associated with obesity and metabolic disorders, which may be related to increased caloric extraction from food, fat deposition and fat production, and impaired insulin sensitivity. In this experiment, the F/B of the HFD group increased significantly (D in Figure 8, p<0.001), while the F/B of the Silymarin80 and Silybin+Iso groups decreased (D in Figure 8, p<0.001, p<0.05). In addition, *Desulfovibrio,* which is usually negatively correlated with body mass index and triglyceride levels and improves MASLD by producing acetate, decreased in the HFD group but did not reach a significant level (E in Figure 8). However, the relative abundance of *Desulfovibrio* increased significantly in each treatment group (E in Figure 8, p<0.01, p<0.05, p<0.01). These results indicated that silymarin can regulate the composition of the intestinal microbiota in HFD-fed mice.

### 2.3 Silymarin regulates the relative abundance of microbial flora producing SCFA or secondary bile acids and intestinal flora with BSH activity

The results of the relative abundance of intestinal flora at the genus level showed that the changes in relative abundance at the genus level caused by HFD were partially restored to ND levels by each treatment group (especially the Silymarin80 group) (A and B in Figure 9). In addition, 80 mg/kg of silymarin significantly increased the relative abundance of bacteria (i.e., *Allobaculum, Bifidobacterium,* and *Dubosiella*) that produce short-chain fatty acids (SCFAs), thereby playing anti-inflammatory, anti-cancer, and reducing intestinal permeability to maintain homeostasis (C in Figure 9). Bacteria with the ability to produce secondary bile acids (i.e., *Clostridium* and *Eubacterium,* both of which belong to the Firmicutes phylum) were also significantly increased in the Silymarin80 group (D in Figure 9), however, the relative abundance of bacteria with bile salt hydrolase (BSH) activity showed inconsistent trends among the groups, indicating that only certain bile acids, but not all bile acids, may play a role in the treatment of MASLD by silymarin. These results indicated that silymarin can modulate the abundance of gut microbiota at the genus level to improve HFD-induced hepatic steatosis.

### 2.4 Silymarin regulates bile acid metabolism and reduces the increase in 7-KDCA levels caused by HFD

To further investigate which bile acids play an important role in improving hepatic steatosis, based on Section 1.2, fecal samples of mice in the ND, HFD, and Silymain80 groups collected 8 weeks after intragastric administration were subjected to bile acid targeted metabolomics analysis. 4 samples for each group, and each sample was a mixture of feces from 2 mice in the group, with a sample of 30 mg (fecal samples were sent to APTBIO for bile acid targeted metabolomics analysis). The results showed that HFD changed the levels of different types of bile acids in mice feces, while Silymarin80 restored the concentrations of some bile acids to ND levels (A in Figure 10). In the ND group, the bile acid with the highest concentration was 12-KLCA, followed by DCA, ApoCA, HDCA, α-MCA, LCA, and 7-KDCA (B in Figure 10). HFD significantly increased the concentrations of 7-KDCA and α-MCA (A in Figure 10, p < 0.001, p < 0.05), and decreased the concentration of TCDCA (A in Figure 10, p < 0.05). After treatment with 80 mg/kg silymarin, the concentration of 7-KDCA decreased to ND levels (A and C in Figure 10, p < 0.001), but the concentrations of α-MCA and TCDCA did not return to ND levels. These results indicated that silymarin may exert its effects by regulating 7-KDCA in bile acids.

### 2.5. FXR is a potential target of 7-KDCA

Farnesoid X receptor (FXR) is an endogenous ligand of bile acid. Therefore, based on Section 1.2, the expression level of FXR in the liver of mice given intragastric administration for 8 weeks was detected by Western blot. The results showed that the expression levels of FXR in the Silymarin30, Silymarin 80 and Silybin+Iso groups were significantly increased (A and B in Figure 11, p<0.001, p<001, p<0.01). In the results of bile acid targeted metabolic analysis, the concentration of 7-KDCA increased in the HFD group and decreased to the ND level in the Silymarin80 group. Therefore, it is speculated that 7-KDCA can negatively feedback regulate FXR, thereby regulating its downstream signal pathways and improving MASLD. To verify this hypothesis, based on Section 1.2, proteins were extracted from the liver samples of mice in the ND group (see Section 2.1 for the extraction method) to obtain a mice liver protein solution; after measuring the protein concentration of the mice liver protein solution with a BCA kit (purchased from CoWin Biotech), the mice liver protein solution was diluted with PBS buffer to a protein concentration of 2 µg/µL to obtain a dilution; the dilution was dispensed into Ep tubes (100 µL per tube) and different concentrations (0 µM, 0.001 µM, 0.003 µM, 0.01 µM, 0.03 µM, 0.1 µM, 0.3 µM) of 7-KDCA (purchased from Sigma-Aldrich) were added to the dilution, and then incubated in a 37°C incubator for 3 h (setting a blank control) for subsequent CETSA and DARTS experiments. For CETSA, the incubated protein solution was heated at 66°C for 3 min and then centrifuged (4°C, 12,000 rpm, 20 min). The supernatant was collected and 5X SDS-PAGE loading buffer was added to the supernatant at a volume ratio of supernatant: buffer = 4:1. The supernatant was heated and denatured at 100°C for 10 min to obtain the test sample. The test sample was subjected to Western blot analysis to detect the expression of FXR and β actin. For DARTS, 1.5% (w/w) pronase enzyme (purchased from MCE) was added to the incubated protein solution (TNC buffer was added to the blank control group), and the solution was incubated at room temperature (25°C) in the dark for 25 minutes to obtain an incubation solution; 5XSDS-PAGE loading buffer was added to the incubation solution at a volume ratio of incubation solution: buffer = 4:1, and the solution was heated at 100°C for 10 minutes to obtain a sample to be tested; the sample to be tested was subjected to Western blot analysis to detect the expression of FXR and βactin. The results showed that 7-KDCA could enhance the thermal stability of FXR in a dose-dependent manner when heated at 66°C (C and D in Figure 11, p<0.05, p<0.01, p<0.01). At the same time, 1.5% (w/w) pronase enzyme can degrade FXR and β actin, while 7-KDCA can dose-dependently inhibit the degradation of FXR by pronase enzyme (E and F in Figure 11, p<0.01, p<0.001, p<0.01). These results indicated that 7-KDCA can bind to FXR and affect the stability of FXR, indicating that FXR is a potential target of 7-KDCA.

In summary, the above experimental results show that silymarin mainly produces its therapeutic effect on MASLD by targeting and regulating the intestinal microbial metabolites LPS and 7-KDCA to bind to TLR4 and FXR receptors and regulate downstream signal pathways. The above research results provide new insights into the mechanism of action of silymarin in the treatment of MASLD and clarified the potential role of 7-KDCA in MASLD.

Obviously, the above-mentioned embodiments are only examples for clear description, and are not intended to limit the implementation manner. For those of ordinary skill in the art, changes or modifications in other different forms can also be made on the basis of the above description. There is no need and cannot be exhaustive of all implementations here. And the obvious changes or changes derived from this are still within the protection scope of the present application.

## Claims

1. Use of a pharmaceutical composition in the preparation of a medicament, wherein the medicament has any one of the following functions:
(a) prevention and/or treatment of fatty liver disease;
(b) anti-inflammatory; and
(c) prevention and/or treatment of neurodegenerative disease;
the active ingredient of the pharmaceutical composition comprises a 7-KDCA regulator; the 7-KDCA regulator comprises silymarin, or the 7-KDCA regulator comprises silybin, silychristin, silydianin and/or isosilybin; the silybin comprises silybin A and/or silybin B; and the isosilybin comprises isosilybin A and/or isosilybin B.

2. The use according to claim 1, wherein the 7-KDCA regulator is silymarin, or the 7-KDCA regulator consists of silybin and isosilybin A; and the silybin consists of silybin A and silybin B.

3. The use according to claim 2, wherein the mass ratio of silybin to isosilybin A in the medicament is (7 to 8):1.

4. The use according to any one of claims 1 to 3, wherein the fatty liver disease comprises metabolic dysfunction associated fatty liver disease.

5. The use according to any one of claims 1 to 4, wherein the treatment of fatty liver disease comprises treating fatty liver disease by regulation of bile acid metabolism; the regulation of bile acid metabolism comprises reducing the binding of 7-KDCA to the farnesol X receptor by lowering the level of 7-KDCA, thereby affecting the stability of the farnesol X receptor.

6. The use according to any one of claims 1 to 5, wherein the treatment of fatty liver disease comprises alleviation of inflammation of patients with fatty liver disease, reduction of liver weight of patients with fatty liver disease, reduction of fat weight of patients with fatty liver disease, reduction of liver weight/body weight ratio of patients with fatty liver disease, improvement of liver fatty degeneration of patients with fatty liver disease, reducing lipid droplet accumulation of patients with fatty liver disease, improving serum indicators of patients with fatty liver disease, improvement of liver function indicators of patients with fatty liver disease and/or improvement of intestinal flora of patients with fatty liver disease.

7. The use according to claim 6, wherein the alleviation of inflammation of patients with fatty liver disease comprises alleviation of inflammation of patients with fatty liver disease by regulating the TLR4/NF-κB signal pathway; the improvement of intestinal flora of patients with fatty liver disease comprises regulating the relative abundance of intestinal flora that produces short-chain fatty acids in the intestine of patients with fatty liver disease, regulating the relative abundance of intestinal flora that produces secondary bile acids in the intestine of patients with fatty liver disease and/or regulating the relative abundance of intestinal flora that contains bile salt hydrolase activity in the intestine of patients with fatty liver disease.

8. The use according to claim 1, wherein the treatment of neurodegenerative disease comprises treating neurodegenerative disease with the medicament as a nerve growth factor-like active substance.

9. A nerve growth factor-like active substance, wherein the ingredient of the nerve growth factor-like active substance comprises a 7-KDCA regulator; the 7-KDCA regulator comprises silymarin, or the 7-KDCA regulator comprises silybin, silychristin, silydianin and/or isosilybin; the silybin comprises silybin A and/or silybin B; and the isosilybin comprises isosilybin A and/or isosilybin B.

10. Use of a pharmaceutical composition in the preparation of a nerve growth factor active substance, wherein the ingredient of the nerve growth factor-like active substance comprises a 7-KDCA regulator; the 7-KDCA regulator comprises silymarin, or the 7-KDCA regulator comprises silybin, silychristin, silydianin and/or isosilybin; the silybin comprises silybin A and/or silybin B; and the isosilybin comprises isosilybin A and/or isosilybin B.
